# EUROPEAN PATENT SPECIFICATION

(11) **EP 2 652 652 B1**
(45) Date of publication and mention of the grant of the patent: **20.11.2019**
(21) Application number: 11797255.4
(22) Date of filing: 16.12.2011
(51) Int. Cl.: G16H 20/17, A61M 5/24, A61M 5/172, A61M 5/315

(54) **MEDICAMENT ADMINISTRATION**
VERABREICHUNG VON MEDIKAMENTEN
ADMINISTRATION DE MÉDICAMENT

(30) Priority: 17.12.2010 EP 10195717
(43) Date of publication of application: 23.10.2013
(60) Divisional application: 19192784.7
(73) Proprietor: Sanofi-Aventis Deutschland GmbH, 65929 Frankfurt am Main (DE)
(72) Inventor: DEBERADINE, Robert, F-75013 Paris (FR)
(74) Representative: McDougall, James
(86) International application number: PCT/EP2011/073086
(87) International publication number: WO 2012/080481

(56) References cited:
- WO-A2-2008/002358
- US-A1- 2002 038 392
- US-A1- 2003 055 685
- US-A1- 2004 260 234
- US-A1- 2006 030 816
- US-A1- 2007 186 923
- US-A1- 2009 030 366
- US-A1- 2009 243 833

## Description

### Field

This invention relates generally to medicament administration, and in particular it relates to a system comprising a medicament administration device, to a method of operating such a system, to a medicament administration device, and to a user token device. Background

### Background

It is known for users to be able to self administer medicaments. Using hypodermic syringes, however, involves some skill and is not suitable for many users. Insulin pens are known to be used for allowing sufferers of diabetes to self inject with insulin. The generic term for such devices is injection pen, and indeed they are often used for other types of medicament including treatments for arthritis, anaemia, anaphylaxis etc. Many different types of injection pen are available on the market, and it is anticipated that some users may be confused and be unable to determine easily which of a number of injection pens available to them contains the correct medicament for a given instance.

US2009243833 discloses a patient care monitoring system and method employing active RFID devices integrated with digital processing, memory and timing circuity for patient identification, care giver identification and for identification of each prescribed treatment procedure, medication and general and/or section care action. At the point-of-care, each care action identity device will match directly with the targeted patient identity device or issue an error warning to prevent mistakes.

US2009030366 discloses a self-injection system that allows a user to inject a drug from a cartridge carrying unique identification information, into any one of a plurality of injection sites. A scanner reads the identification information of the cartridge and cooperates with a central processing unit to determine the validity of the drug in order to permit an injection procedure to commence. The central processing unit has a memory for storing the different injection parameters and controls a drive unit for driving fluid from the cartridge and through a needle into the selected tissue, at the injection parameter that is associated with the user selected tissue for the injection.

US 2002038392 discloses controlling IV medication delivery and monitoring, by providing information tags on IV bags that specify delivery parameters, obtaining delivery parameters for at least on bag, associating a controller with a particular patient, comparing patient information for the particular patient with the delivery parameters, determining the efficacy of delivering the medicament to the patient and affecting pump control as a function of the comparison.

US20070186923 discloses a dispensing device that provides a means for detecting the identity of the patient, a lock-out feature and a means to prevent unauthorised access to stored drugs.

### Summary

The invention is defined by the appended claims.

### Brief Description of the Drawings

Embodiments will now be described, by way of example only with reference to the accompanying drawings in which:
Figure 1A is a schematic perspective view of a user token device, in the form of a bracelet, according to aspects of the invention;
Figure 1B is a circuit diagram of components of the bracelet of Figure 1A;
Figure 2 is a schematic diagram illustrating apparatus that is usable to program the bracelet of Figures 1A and Figure 1B.
Figure 3 is a schematic part cross-sectional drawing of a medicament administration device in the form of an injector pen according to a first embodiment;
Figure 4 is a similar view of an injection pen according to a second embodiment;
Figure 5 is a similar view of an injector pen according to a third embodiment; and Figures 6 to 9 are flowcharts illustrating operation of the bracelet of Figures 1A and 1B and the injector pen of one of Figures 3 to 5 according to the first to fourth embodiments.

### Detailed Description of the Embodiments

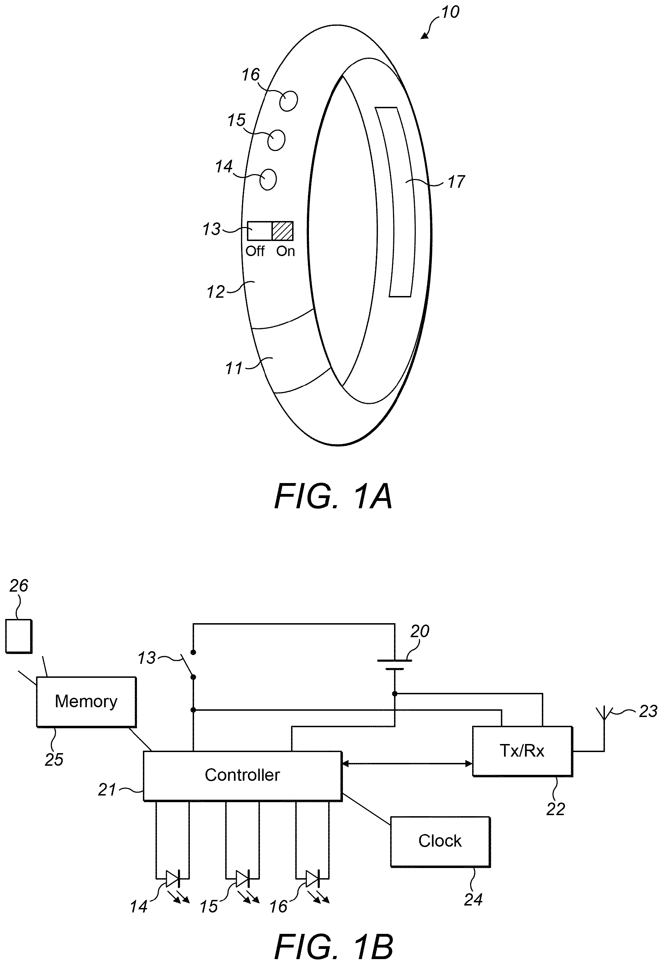

Figure 1A illustrates a user token device 10. Here, the device 10 is a bracelet having a generally toroidal shape, although the innermost surface of the toroidal shape generally takes the shape of the surface of a cylinder. The shape of the bracelet 10 allows it to be worn around the wrist of a user. The bracelet 10 optionally includes a clasped 11, by which the bracelet 10 can be opened and closed so as to facilitate easy removal etc. Aside from the clasp 11, the bracelet 10 comprises a main body 12 and a number of components, some of which are shown in Figure 1A and others which are shown in Figure 1B.

As shown in Figure 1A, the bracelet 10 may include a switch 13, for example a slider switch, which is movable by a user between on and off positions. In Figure 1A, the on position is shown as being to the right, and the off position to the left. Also provided on an externally facing surface of the bracelet 10 are first to third indicators 14 to 16.

In this example, the indicators 14 to 16 are illuminating indicators. Here, they are in the form of light emitting diodes (LEDs). In this example, the first LED 14 is coloured green, the second LED 15 is coloured yellow and the third LED 16 is coloured red. Alternatively, at least one multi-coloured LED may be used. The indicators 14 to 16 may instead be replaced by any visual, audio, vibratory etc. indicator.

On an internal surface of the bracelet 10 is a label 17. This may be provided with information identifying a user that is associated with the bracelet 10. The label may for instance include the user's name and patient number, and may optionally include a date of birth.

Contained within the body 12 of the bracelet 10 are a number of electronic components. These are shown in Figure 1B. In particular, a battery 20 is shown as forming part of an electrical circuit in series with the switch 13. When the switch 13 is closed, i.e. in the on position in Figure 1A, electrical power from the battery is provided to a controller 21 and to a transceiver 22, which includes a transmitter and a receiver, and is in turn connected to an antenna at 23. The controller is also connected to a clock 24 and to a memory 25. The first to third LEDs 14 to 16 are connected to outputs of the controller 21, and are able selectively to be switched on and off thereby.

The controller 21 is in bidirectional communication with the transceiver 22. Computer program software 26 residing in the memory 25 is the basis for operation of the controller 21. Put another way, the controller 21 operates under control of the instructions formed by the software at 26, which is stored in the memory 25.

The bracelet 10 is able to receive information via the antenna 23 in the transceiver 22 and storage in the memory at 25. The bracelet 10 also is able to communicate with an external medicament administration device, and to illuminate relevant ones off the first and third LEDs 14 to 16 dependent at least in part on information received from the medicament administration device. The bracelet 10 also is, in some embodiments, configured to send information from the memory 25 to the medicament administration device. In some embodiments, the bracelet 10 is configured to send information that controls whether or not a medicament administration device delivers a medicament to a user.

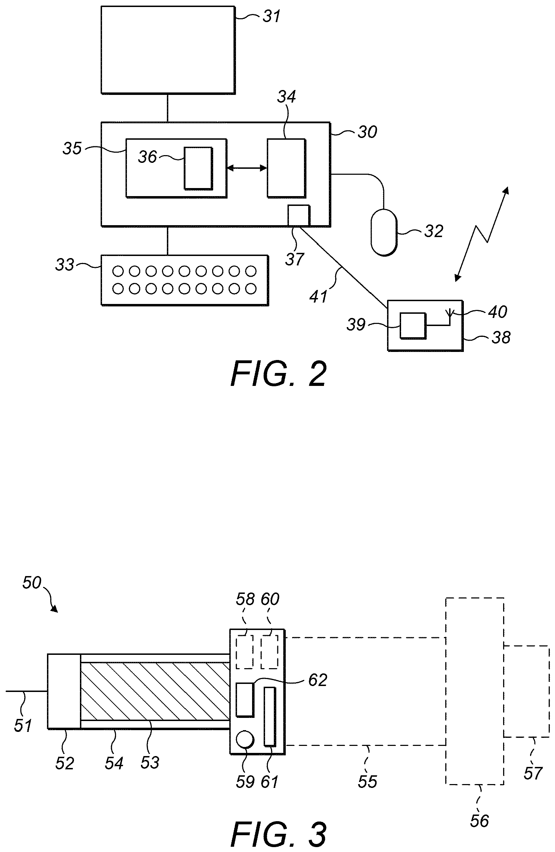

Figure 2 shows an apparatus that may be used to program the bracelet 10. The apparatus at Figure 2 comprises computing apparatus, in particular a computer 30, which is connected to a display device, in the form of a monitor 31, and to input devices in the form of a mouse 32 and keyboard 33. The computer 30 includes a processor 34 which is in bidirectional communication with a memory 35, in which is stored one or more computer programs 36.

The computing device 30 includes an interface 37 to an external device. The interface 37 may, for instance, be a wired interface, for example a universal serial bus (USB) port. An external device 38 is shown as including a transceiver 39 and an antenna 40. The external device may be termed a key, and may be relatively small in size. The external device 38 is connected to the USB port 37 by a USB cable 41. Alternatively, the transceiver 39 and antenna 40 may reside within the computing device 30.

The computing device 30, in particular the processor 34 thereof, operates under control of the computer programs 36 that are stored in the memory 35. The computer programs allow a medical professional to create a record consisting of information identifying a user, such as a patient, and information relating to medicaments associated with a user. The information relating to medicaments may merely identify one or more medicaments. The information may in addition identify a dosage regimen for the medicament. The regimen may be in terms of doses and times of day or intervals between doses. The information relating to one or more medicaments may identify a cartridge type, the cartridge type relating to a type that is used to contain the corresponding medicament. The information may relate only to one medicament or it may relate to two or more medicaments that are to be administered to the user/patient. The computer programs 36 are constructed so as to allow the medical professional to complete the record and to view the record on the monitor 31.

The programs 36 are configured also to allow the medical professional to program the bracelet 10 with information from that record. Programming occurs by the medical professional or the user/patient moving the switch 13 to the on position and by the placing of the key 38 in close proximity to the bracelet 10. When these conditions are satisfied, the bracelet 10 may communicate with the computing apparatus 30 via the transceivers 22, 39 and the antennas 40, 23. This may occur in any convenient manner. For instance, the bracelet 10, in particular the controller 21 thereof, may be configured upon first receiving electrical power from the battery 20 to transmit an announcement signal via the transceiver 22 and the antenna 23. On receiving the announcement signal through the antenna 40 and the transceiver 49, the computing apparatus may then perform a handshaking procedure or other procedure so as to establish communication with the bracelet 10. The handshaking procedure may comprise an authentication and/or authorization procedure. Authentication and/or authorization may comprise entry of a PIN code on the keyboard 33 of the computing device 30. In this way, unauthorized modification of the patient record may be prevented, so that only for example the medical professional may transmit a modified patient's dosage regimen to the bracelet.

After communication is established, the computing apparatus 30 may request that the controller 21 of the bracelet accepts being programmed with the record. Assuming that this is permitted, as determined by the controller 21 operating under control of the computer program 26, the bracelet sends an acknowledgement message to the computing apparatus 30. Following receipt of the acknowledgement, the computing apparatus 30 transmits information relating to medicaments pertaining to the user/patient to the bracelet 10, where the controller 21 causes the information to be stored in the memory 25.

In this way, the bracelet 10 has been programmed with information relating to medicaments of the user/patient by the medical practitioner, through control of the computing apparatus 30. By wearing the bracelet 10, the user/patient then always has the information relating to their medicaments with them wherever they go.

Figure 3 is a medicament administration device in the form of an injection pen 50. Components of the injection pen 50 include a needle 51, a pen needle support 52, a cartridge 53, a lower housing part 54, an upper housing part 55, a dose selector 56 and an activator 57.

The injection pen 50 also includes a transceiver 58, which includes a transmitter and receiver (not shown). The injection pen 50 may also include an indicator 59, which may be an LED. A cartridge detector/identifier 60 may take one of a number of different forms. In one form, it may simply detect the presence of the cartridge 53 within the injection pen 50. Alternatively, it may detect an identity of the cartridge 53. Cartridge identity detection may occur in one of a number of different ways, for example a special form or size of a cartridge, a mechanical identification, an opto-electronic identification, such as through a bar-code reader, an electronic identification ,such as an RF-ID tag and reader, and/or the like..

A controller 62 is coupled to the transceiver 58, the LED 59 and the cartridge detector/identifier 60.

The injection pen 50 optionally includes a cartridge size adjuster 61. This is operable by the user/patient to set a cartridge size. The cartridge size set using the cartridge size adjuster 61 determines the size of cartridge 53 that can be inserted into the injection pen 50. The cartridge size adjuster 61 may include a screw thread mechanism for instance.

To include a new cartridge 53 into the injection pen 50, a user may separate the lower housing part 54 from the upper housing part 55. This may be performed by an unscrewing movement, utilising a screw thread mating between the two housing parts 54, 55. When the upper housing part 55 is removed, the components 58 to 62 remain coupled to the lower housing part 54 and provide an aperture for insertion of the cartridge 53. Adjustment of the cartridge size adjuster 61 by the user/patient adjusts the size and/or shape of the aperture, and thus permits only certain cartridges to be inserted into the lower housing part 54. The cartridge size adjuster 51 may be configured such as to impose a maximum diameter on cartridges that may be inserted into the lower housing part 52, in which case all cartridges of a smaller diameter can be inserted. Alternatively, the cartridge size adjuster 61 may be configured so as to allow only cartridges of a size that is set by the user/patient to be inserted into the lower housing part 54, with cartridges of other sizes (both larger and smaller diameter) being prevented from being inserted into the lower housing part 54.

The controller 62 is able to communicate with the controller 21 of the bracelet 10 through the transceiver 58 and the transceiver 22. Communication may be unidirectional, from the controller 62 to controller 21. Alternatively, communication may be bi-directional, i.e. occur in both directions between the controller 62 and the controller 21. The controller 62 communicates to the bracelet 10 information provided by the cartridge/identifier. This information may simply indicate whether or not the cartridge is present, which may be detected mechanically, optically, electrically etc. Alternatively, the information may communicate the cartridge type. This may be detected by the controller 62 through detection of some identifying characteristic of the cartridge 53 itself. This may take the form of a barcode, a sigil, a conductive pattern, etc., or some physical characteristic such as size, opacity, etc. Alternatively, the information may be derived from the setting of the cartridge size adjuster 61 since the size of the cartridge 53 is dependent upon this setting.

The transceivers 22 and 58 may be able to communicate through any suitable short-range protocol, either proprietary or standardised. The maximum range of communication may be limited by the transceivers, for instance to a maximum of 10 m, 5 m, 1 m or 10 cm. The transceivers 22 and 58 may use Bluetooth™, Zigbee™, RFID, IrDA™ or some other suitable protocol.

Figure 3 illustrates two separate embodiments. In the first embodiment, the cartridge size adjuster 61 is absent. In the second embodiment, the cartridge size adjuster 61 is present.

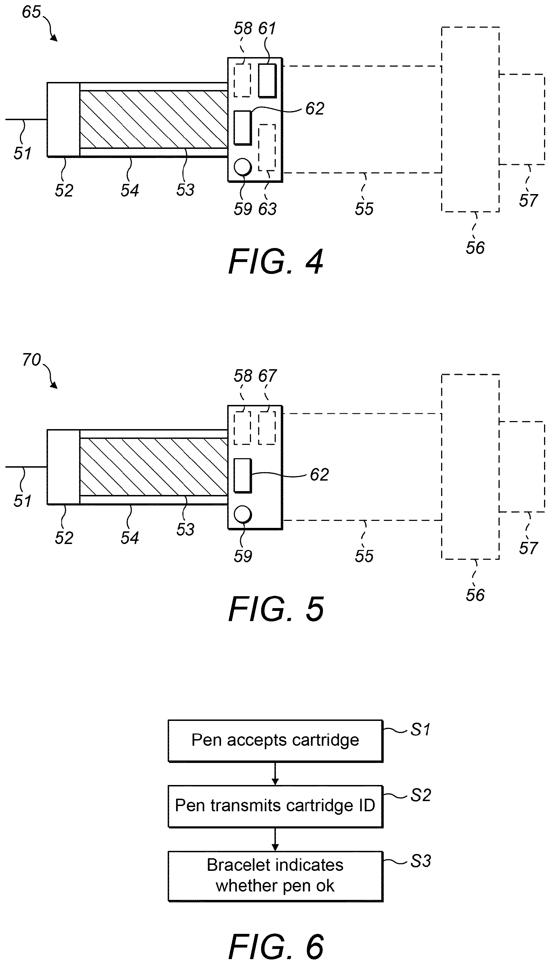

Operation of the first embodiment will now be described with reference to Figure 6.

Referring to Figure 6, the injection pen 50 first accepts the cartridge 53 at step S1. This involves the user separating the first lower and upper housing parts 54, 55 and inserting the cartridge 53 into the aperture thereby formed. In this embodiment, no cartridge size adjuster 61 is present. In this embodiment, the size of the cartridge is not used to determine the cartridge type, nor the contents of the cartridge. Instead, the cartridge type and/or medicament contained within the cartridge is determined by the controller 62 and the cartridge detector/identifier 60 by analysing some aspect of the cartridge 52, for instance a bar code or other identifier attached thereto, or by analysing some aspect of the medicament, e.g. through the use of a pH sensor associated with the cartridge 53 and in contact with the medicament..

At step S2, the injection pen 50 transmits the identifier pertaining to the cartridge 53 to the bracelet 10. This message is received by the bracelet 10. The bracelet 10, in particular the controller 21 thereof, compares the cartridge identifier received from the injection pen 50 to the medicament information stored in the memory 25. On the basis of the comparison, the controller 21 determines whether the cartridge identifier is consistent with the medicament information.

At step S3, the bracelet 10 indicates to the user whether the injection pen 50 is okay to use, on the basis of the determination. In the event of a positive determination, the controller 21 may illuminate the green LED 14. In the event of a negative determination, i.e. in determining that the injection pen 50 is not suitable for the user/patient, the controller 21 may illuminate the red LED 16, or alternatively cause the red LED to flash. If a determination cannot be made for any reason, this may be indicated to the user by the controller 21 illuminating the yellow LED 50. On seeing the yellow LED illuminated, the user would know to check the injection pen 50, particularly to ensure that the cartridge is installed properly and that it is in sufficient close proximity to the bracelet 10 to enable communication therebetween.

Optionally, the bracelet 10 sends the result of the determination to the injection pen 50 through transceivers 22 and 58. The LED 59 of the injection pen 50 may indicate to a user/patient the result of the determination, for example by lighting up in the same colour as one of the LEDs 14, 15, 16 of bracelet 10.

The operation of the bracelet 10 and the injection pen 50 described above may result in the user being presented with a warning to prevent them from administering medicaments other than in accordance with the record information that is programmed into the memory 25 of the bracelet 10.

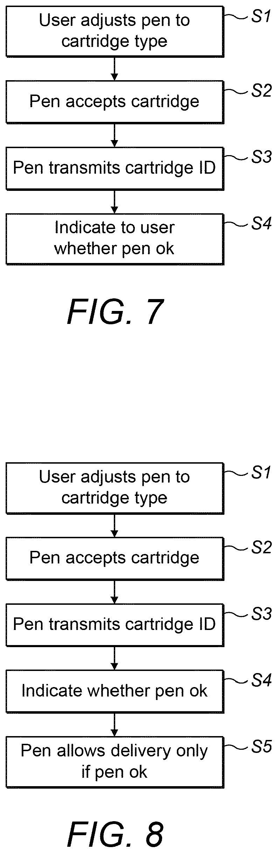

A second embodiment will now be described with reference to Figures 3 and 7. In this embodiment, the cartridge size adjuster 61 is present in the injection pen 50.

At step S1, the user adjusts the injection pen to accept the desired cartridge size. This is achieved by the user manipulating the cartridge size adjuster 61 until a desired value or other indicator is visible at a relevant part of the exterior of the injection pen 50. The indicator may be a number, for instance "5", or alternatively may name a medicament, for instance "insulin". The user then proceeds to load the cartridge 53 into the lower housing part 54, in the same way as described above. Assuming the cartridge 53 is of the correct size, the injection pen 50 receives the cartridge at step S2.

The injection pen 50, and in particular the controller 62 thereof, then detects the type or identifier that is associated with the cartridge. This may occur as described above in relation to the first embodiment. Alternatively this may occur by detecting the setting of the cartridge size adjuster 61. The cartridge identifier or cartridge type is transmitted to the bracelet 10 by the injection pen at step S3. The bracelet then performs as recited above in relation to the first embodiment and at step S4 indicates to the user whether or not the injection pen 50 is okay for use by the user/patient.

The third embodiment will now be described with reference to Figure 4 and the flow chart of Figure 8.

Figure 4 shows an injection pen 65. Reference numerals are retained from Figure 3 for like elements. The pen 65 also includes a delivery enabler/preventer 63.

At step S1 of Figure 8, the user/patient adjusts the cartridge size adjuster 61 of the insulin pen 50 to the required cartridge type. This is performed as discussed above in relation to the second embodiment. At step S2, the insulin pen 50 accepts the cartridge 53. Again this is as described in relation to the first and second embodiments. At step S3, the insulin pen 50 transmits the identifier of the cartridge or the cartridge type to the bracelet 10. This is as discussed above in relation to the first embodiment or the second embodiment. The bracelet 10 then determines whether or not the cartridge 53, and the contents thereof, are consistent with the medicament information stored in the memory 25. At step S4, the user is provided with an indication as to whether the injection pen 65 is suitable for use by the user/patient. This may occur by controlling the first and third LEDs 14 to 16 as described above. Alternatively or in addition, this may occur by control of the LED 59. This may occur through the controller 21 of the bracelet transmitting information to the controller 62 of the injection pen 65 indicating whether or not the cartridge 53 is consistent with the medicament information stored in the memory 25. This information is also used by the injection pen 65 to control whether or not the medicament is delivered to the user/patient as is described below. At step S4, the controller 62 may cause the LED 59 to flash quickly if the injection pen 65 is indicated as not being suitable by the user/patient and to be illuminated constantly or/slowly if the injection pen 65 is suitable for use by the user/patient.

At step S5, the injection pen 65 allows delivery of the contents of the cartridge 53 only if the information received from the bracelet 10 indicates that the cartridge 53, and thus the contents thereof, are suitable for use by the user/patient in accordance with the medicament regimen stored in the memory 25. This is effected by the delivery enabler/preventer 63 either preventing or allowing operation of the activator 57 by the user/patient to result in the injection of the contents of the cartridge 53 through the needle 51. The enabler/preventer 63 may allow delivery of the medicament in accordance with the dosage regimen for the medicament prescribed for the user/patient. For example, the enabler/preventer 63 may prevent selection of a dose if a cartridge containing a wrong medicament is inserted. Alternatively or in addition, the enabler/preventer 63 may prevent selection of a dose outside a dosage window (for example a window of 10 to 20 units) in accordance with the dosage regimen for the medicament for the user/patient, but allow selection of a dose within the dosage window. Further, the enabler/preventer 63 may enable selection of a dose only after a certain time since the last administration of a dose of the medicament for example after at least 20 hours, and prevent selection of a dose otherwise. Enabling or preventing administration of a dose may occur in any suitable way.

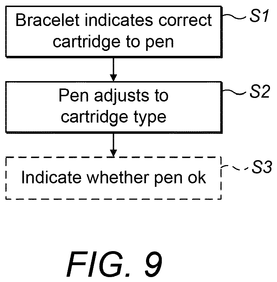

The fourth embodiment will now be described with reference to Figure 5 and the flow chart of Figure 9. Reference numerals are retained from Figures 3 and 4 for like elements. An automatic cartridge size adjuster 67 is not manually operable, but instead is automatically operable by the injector pen 70.

At step S1, the bracelet 10 indicates the correct cartridge identifier or cartridge type to the injection pen 70. Following receipt of this information by the injection pen 70, the controller 62 causes the cartridge size adjuster 67 to be adjusted such as to accommodate the corresponding cartridge. This may prevent cartridges of any other type being inserted into the injection pen 70, or it may allow only cartridges which have a smaller dimension and the relevant cartridge types being inserted, as described above with reference to the second embodiment.

Following this step, the user/patient may insert a cartridge into the aperture that leads to the interior of the lower housing part 54. If the user attempts to insert the wrong cartridge at this stage, this will be prevented by the cartridge size adjuster 67. The cartridge detector/identifier 60 then detects that the cartridge 53 is in place. Correct placement of the cartridge may be indicated to the user through illumination of the LED 59 by the controller 62.

At step S3, which is optional, the user is provided with an indication as to whether the injection pen 65 is suitable for use by the user/patient. This may occur by controlling the first and third LEDs 14 to 16 as described above. Alternatively or in addition, this may occur by control of the LED 59. This may occur through the controller 21 of the bracelet transmitting information to the controller 62 of the injection pen 65 indicating whether or not the cartridge 53 is consistent with the medicament information stored in the memory 25.

The user/patient may operate the actuator 57 to effect medicament delivery as required. The injection pen 70 may not impose any restrictions on delivery, as described for the third embodiment.

A method of use by a user is as follows.

The user firstly operates the system so as to cause the medicament administration device and the user token device to communicate wirelessly. This may require merely moving the two devices together, or may involve operating a power switch on one or both devices.

The user then observes an indicator on the medicament administration device and/or the user token device. The indicator indicates whether the medicament is in accordance with the information stored into the user token device.

The user then administers medicament from the medicament administration device only if the indicator indicates that.the medicament is in accordance with the information stored into the user token device.

The term "medicament", as used herein, means a pharmaceutical formulation containing at least one pharmaceutically active compound. In one example, the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the abovementioned pharmaceutically active compound. Alternatively, the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis. Alternatively, the pharmaceutically active compound comprises at least one peptide for the treatment and/or prophylaxis of diabetes mellitus or complications associated with diabetes mellitus such as diabetic retinopathy. Alternatively, the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

## Claims

1. A system comprising a user token device (10) and an injection pen (50,65,70) configured to allow user replacement of a medicament cartridge (53), wherein:
the user token device (10) comprises a memory (25) configured to store information relating to at least one medicament pertaining to a user, wherein said information includes identification of one or more permitted cartridge type,
the injection pen (50,65,70) and the user token device (10) are configured to communicate wirelessly in at least a unidirectional fashion, and
wherein the user token device (10) is configured to transmit information identifying a permitted cartridge type to the injection pen (50,65,70), and
the injection pen (50,65,70) comprises a cartridge size adjuster (61,67), wherein the cartridge size adjuster (61,67) is adjustable to accommodate a cartridge (53) of the permitted cartridge type, and
wherein the injection pen (50,65,70) is configured to prevent use of a cartridge (53) other than a cartridge of the permitted cartridge type transmitted by the user token device (10) in accordance with the information relating to the at least one medicament pertaining to the user stored in the user token device (10).

2. A system as claimed in claim 1, wherein the injection pen (50,65,70) is configured to detect a type of a medicament or cartridge type that is included in the injection pen (50,65,70), and to transmit information identifying the medicament or cartridge type to the user token device (10).

3. A system as claimed in claim 2, wherein the user token device (10) further comprises an indicator (14,15,16) configured to indicate whether the injection pen (50,65,70) may be used in accordance with the detected medicament type.

4. A system as claimed in any preceding claim, wherein the user token device (10) is configured to determine from information received from the injection pen (50,65,70) that the injection pen (50,65,70) is able to be used in accordance with the information relating to medicaments pertaining to the user that is programmed into the user token device (10), and to indicate to the user whether the injection pen (50,65,70) is able to be used in accordance with the information relating to medicaments pertaining to the user.

5. A system as claimed in any preceding claim, wherein the injection pen (50,65,70) and/or the user token device (10) further comprises an indicator (59; 14,15,16) configured to indicate whether the injection pen (50,65,70) may be used in accordance with the stored information.

6. A system as claimed in any of claims 1 to 3, wherein the injection pen (50,65,70) is configured to determine from information received from the user token device (10) that the injection pen (50,65,70) is able to be used in accordance with the information relating to medicaments pertaining to the user that is programmed into the user token device (10), and to indicate to the user whether the injection pen (50,65,70) is able to be used in accordance with the information relating to medicaments pertaining to the user.

7. A system as claimed in claim 1, wherein the injection pen (50,65,70) is configured to prevent administration of medicament contained within the injection pen unless the injection pen (50,65,70) determines the user token device to be in proximity thereto.

8. A system as claimed in claim 7, wherein the injection pen (50,65,70) and/or the medicament cartridge (53) is configured to spoil medicament contained in the medicament cartridge (53) if the injection pen (50,65,70) is damaged.

## Patentansprüche

1. System, umfassend eine Benutzer-Token-Vorrichtung (10) und einen Injektionspen (50, 65, 70), der dazu ausgelegt ist, das Auswechseln einer Medikamentenkartusche (53) durch einen Benutzer zu gestatten, wobei:
die Benutzer-Token-Vorrichtung (10) einen Speicher (25) umfasst, der zum Speichern von Informationen bezüglich zumindest eines Medikaments, das zu einem Benutzer gehört, ausgelegt ist, wobei die Informationen die Identifizierung von einer oder mehreren zulässigen Kartuschenarten umfassen,
der Injektionspen (50, 65, 70) und die Benutzer-Token-Vorrichtung (10) für eine drahtlose Kommunikation auf zumindest unidirektionale Weise ausgelegt sind, und wobei
die Benutzer-Token-Vorrichtung (10) zum Übertragen von Informationen, die eine zulässige Kartuschenart identifizieren, an den Injektionspen (50, 65, 70) ausgelegt ist, und
der Injektionspen (50, 65, 70) ein Kartuschengrößeneinstellmittel (61, 67) umfasst, wobei das Kartuschengrößeneinstellmittel (61, 67) zur Aufnahme einer Kartusche (53) der zulässigen Kartuschenart einstellbar ist, und
wobei der Injektionspen (50, 65, 70) dazu ausgelegt ist, die Verwendung einer anderen Kartusche (53) als einer Kartusche der zulässigen Kartuschenart, die von der Benutzer-Token-Vorrichtung (10) gemäß den Informationen bezüglich des zumindest einen Medikaments, das zu dem Benutzer gehört, die in der Benutzer-Token-Vorrichtung (10) gespeichert sind, übertragen wurde, zu verhindern.

2. System nach Anspruch 1, wobei der Injektionspen (50, 65, 70) dazu ausgelegt ist, eine Art eines Medikaments oder eine Kartuschenart zu erkennen, die im Injektionspen (50, 65, 70) enthalten ist, und Informationen, die die Medikamenten- oder die Kartuschenart identifizieren, an die Benutzer-Token-Vorrichtung (10) zu übertragen.

3. System nach Anspruch 2, wobei die Benutzer-Token-Vorrichtung (10) ferner eine Anzeige (14, 15, 16) umfasst, die dazu ausgelegt ist anzuzeigen, ob der Injektionspen (50, 65, 70) gemäß der erkannten Medikamentenart verwendet werden kann.

4. System nach einem der vorhergehenden Ansprüche, wobei die Benutzer-Token-Vorrichtung (10) dazu ausgelegt ist, anhand der vom Injektionspen (50, 65, 70) empfangenen Informationen zu bestimmen, dass der Injektionspen (50, 65, 70) gemäß den Informationen bezüglich Medikamenten, die zum Benutzer gehören, die in die Benutzer-Token-Vorrichtung (10) einprogrammiert sind, verwendet werden kann, und dem Benutzer anzuzeigen, ob der Injektionspen (50, 65, 70) gemäß den Informationen bezüglich Medikamenten, die zum Benutzer gehören, verwendet werden kann.

5. System nach einem der vorhergehenden Ansprüche, wobei der Injektionspen (50, 65, 70) und/oder die Benutzer-Token-Vorrichtung (10) ferner eine Anzeige (59; 14, 15, 16) umfassen, die dazu ausgelegt ist anzuzeigen, ob der Injektionspen (50, 65, 70) gemäß den gespeicherten Informationen verwendet werden kann.

6. System nach einem der Ansprüche 1 bis 3, wobei der Injektionspen (50, 65, 70) dazu ausgelegt ist, aus den von der Benutzer-Token-Vorrichtung (10) empfangenen Informationen zu bestimmen, dass der Injektionspen (50, 65, 70) gemäß den Informationen bezüglich Medikamenten, die zum Benutzer gehören, die in die Benutzer-Token-Vorrichtung (10) einprogrammiert sind, verwendet werden kann, und dem Benutzer anzuzeigen, ob der Injektionspen (50, 65, 70) gemäß den Informationen bezüglich Medikamenten, die zum Benutzer gehören, verwendet werden kann.

7. System nach Anspruch 1, wobei der Injektionspen (50, 65, 70) dazu ausgelegt ist, die Verabreichung eines im Injektionspen enthaltenen Medikaments zu verhindern, es sei denn, der Injektionspen (50, 65, 70) bestimmt, dass die Benutzer-Token-Vorrichtung sich in dessen Nähe befindet.

8. System nach Anspruch 7, wobei der Injektionspen (50, 65, 70) und/oder die Medikamentenkartusche (53) dazu ausgelegt sind, ein in der Medikamentenkartusche (53) enthaltenes Medikament unbrauchbar zu machen, wenn der Injektionspen (50, 65, 70) beschädigt ist.

## Revendications

1. Système comprenant un jeton d'authentification d'utilisateur (10) et un stylo d'injection (50, 65, 70) configuré pour permettre le remplacement par un utilisateur d'une cartouche de médicament (53), dans lequel :
le jeton d'authentification d'utilisateur (10) comprend une mémoire (25) configurée pour stocker des informations concernant au moins un médicament approprié pour un utilisateur, lesdites informations comportant l'identification d'un ou plusieurs types de cartouche autorisés,
le stylo d'injection (50, 65, 70) et le jeton d'authentification d'utilisateur (10) sont configurés pour communiquer sans fil au moins d'une manière unidirectionnelle, et
dans lequel le jeton d'authentification d'utilisateur (10) est configuré pour transmettre des informations identifiant un type de cartouche autorisé au stylo d'injection (50, 65, 70), et
le stylo d'injection (50, 65, 70) comprend un dispositif d'ajustement à la taille de cartouche (61, 67), le dispositif d'ajustement à la taille de cartouche (61, 67) étant ajustable pour accueillir une cartouche (53) du type de cartouche autorisé, et
dans lequel le stylo d'injection (50, 65, 70) est configuré pour empêcher l'utilisation d'une cartouche (53) différente d'une cartouche du type de cartouche autorisé transmis par le jeton d'authentification d'utilisateur (10) conformément aux informations concernant l'au moins un médicament approprié pour l'utilisateur stockées dans le jeton d'authentification d'utilisateur (10).

2. Système selon la revendication 1, dans lequel le stylo d'injection (50, 65, 70) est configuré pour détecter un type de médicament ou un type de cartouche qui est contenu dans le stylo d'injection (50, 65, 70), et pour transmettre des informations identifiant le type de médicament ou de cartouche au jeton d'authentification d'utilisateur (10).

3. Système selon la revendication 2, dans lequel le jeton d'authentification d'utilisateur (10) comprend en outre un indicateur (14, 15, 16) configuré pour indiquer si le stylo d'injection (50, 65, 70) peut être utilisé conformément au type de médicament détecté.

4. Système selon une quelconque revendication précédente, dans lequel le jeton d'authentification d'utilisateur (10) est configuré pour déterminer, à partir d'informations reçues depuis le stylo d'injection (50, 65, 70), que le stylo d'injection (50, 65, 70) peut être utilisé conformément aux informations concernant des médicaments appropriés pour l'utilisateur qui sont programmées dans le jeton d'authentification d'utilisateur (10), et pour indiquer à l'utilisateur si le stylo d'injection (50, 65, 70) peut être utilisé conformément aux informations concernant des médicaments appropriés pour l'utilisateur.

5. Système selon une quelconque revendication précédente, dans lequel le stylo d'injection (50, 65, 70) et/ou le jeton d'authentification d'utilisateur (10) comprennent en outre un indicateur (59 ; 14, 15, 16) configuré pour indiquer si le stylo d'injection (50, 65, 70) peut être utilisé conformément aux informations stockées.

6. Système selon l'une quelconque des revendications 1 à 3, dans lequel le stylo d'injection (50, 65, 70) est configuré pour déterminer, à partir d'informations reçues depuis le jeton d'authentification d'utilisateur (10), que le stylo d'injection (50, 65, 70) peut être utilisé conformément aux informations concernant des médicaments appropriés pour l'utilisateur qui sont programmées dans le jeton d'authentification d'utilisateur (10), et pour indiquer à l'utilisateur si le stylo d'injection (50, 65, 70) peut être utilisé conformément aux informations concernant des médicaments appropriés pour l'utilisateur.

7. Système selon la revendication 1, dans lequel le stylo d'injection (50, 65, 70) est configuré pour empêcher l'administration d'un médicament contenu dans le stylo d'injection sauf si le stylo d'injection (50, 65, 70) détermine que le jeton d'authentification d'utilisateur est à proximité de celui-ci.

8. Système selon la revendication 7, dans lequel le stylo d'injection (50, 65, 70) et/ou la cartouche de médicament (53) sont configurés pour détériorer un médicament contenu dans la cartouche de médicament (53) si le stylo d'injection (50, 65, 70) est endommagé.
